(19) Europäisches Patentamt
European Patent Office
Office européen des brevets

(11) Publication number: 0 074 711 B1

(12) **EUROPEAN PATENT SPECIFICATION**

(45) Date of publication of patent specification: 15.01.86

(51) Int. Cl.⁴: C 07 D 405/06, C 07 D 405/04, A 61 K 31/415, A 61 K 31/335

(21) Application number: 82304072.0

(22) Date of filing: 02.08.82

(54) **Benzodioxane-imidazoline compounds, their preparation and use.**

(30) Priority: 03.08.81 US 289679

(43) Date of publication of application: 23.03.83 Bulletin 83/12

(45) Publication of the grant of the patent: 15.01.86 Bulletin 86/03

(84) Designated Contracting States: AT BE CH DE FR GB IT LI LU NL SE

(56) References cited:
EP-A-0 033 655
EP-A-0 047 531
US-A-2 979 511
US-A-4 315 021

(73) Proprietor: SYNTEX (U.S.A.) INC.
3401 Hillview Avenue
Palo Alto, California 94304 (US)

(72) Inventor: Kluge, Arthur Frederick
1683 Parkhills Avenue
Los Altos California 94022 (US)
Inventor: Strosberg, Arthur Martin
120 Lucero Way
Portola Valley California 95025 (US)
Inventor: Whiting, Roger Lewis
98 Colinton Road
Edinburgh EH14 4AS (GB)
Inventor: Christie, George Allan
Boag's Mill Bogsmill Road
Colinton Dell Edinburgh EH14 2LX (GB)

(74) Representative: Armitage, Ian Michael et al
MEWBURN ELLIS & CO. 2/3 Cursitor Street
London EC4A 1BQ (GB)

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 074 711 B1

**Description**

The present invention is concerned with compounds, compositions and methods useful for treating symptomologies in human beings which are effected by $\alpha_2$ blockade.

Large numbers of compounds are known which affect various physiological systems related to synaptic control. The class most closely related to the compounds of the present invention is that disclosed in U.S. 2,979,511, issued April 11, 1961 to Krapcho, et al. This patent purports to disclose, generically, compounds of and related to the present invention, specifically those of formula I' wherein n is equal to 0 or 1, the R groups are hydrogen or lower alkyl, and $R^1$ is hydrogen.

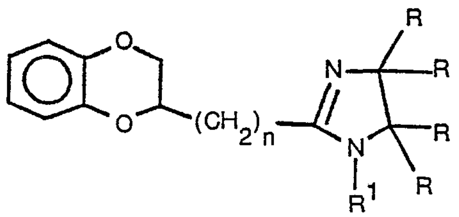

(I')

Also, specifically claimed in the Krapcho patent are 2 - (1,4 - benzodioxan - 2 - yl) - imidazoline, and its hydrochloride. These compounds are disclosed as peripherally acting vasodilators. The methods of preparation given therein, do not, in our hands, yield the imidazoline ring, but rather the open chain counterparts.

The present invention concerns specifically 2 - (1,4 - benzodioxan - 2 - ylmethyl) imidazoline of the following formula

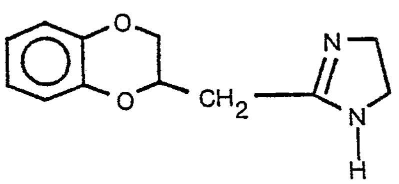

(I)

and the pharmaceutically acceptable acid addition salts thereof.

These compounds have been shown to block $\alpha_2$ receptors in pithed rats, hence in two other aspects the invention concerns compounds hereof for pharmaceutical use, especially for affecting physiological phenomena related to $\alpha_2$ control in human beings, and compositions for this purpose containing these compounds. In a fourth aspect, the invention is directed to methods of preparing these compounds.

Definitions:

As used herein:

"Pharmaceutically acceptable acid addition salt" refers to those salts which retain the biological effectiveness and properties of the free bases and which are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid and the like.

"Optional" or "optionally" means that the subsequently described event or circumstance may or may not occur, and that the description includes instances where said event or circumstance occurs and instances in which it does not. For example, "optionally followed by converting the free base to the acid addition salt" means that said conversion may or may not be carried out in order for the process described to fall within the invention, and the invention includes those processes wherein the free base is converted to the acid addition salt and those processes in which it is not.

The compound of the invention is prepared according to Reaction Scheme I.

REACTION SCHEME I

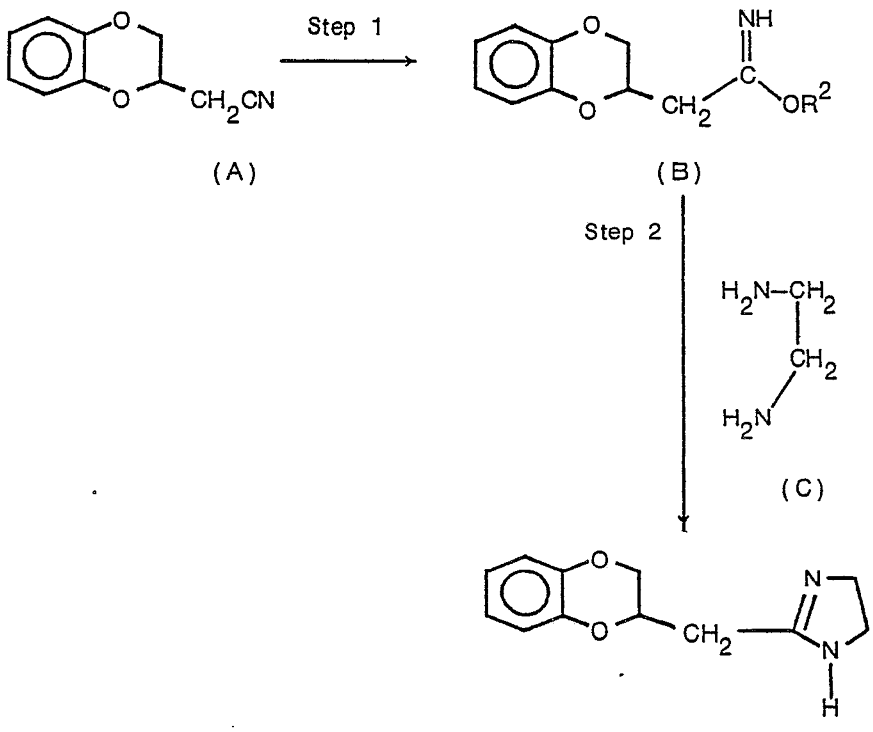


(A) (B) (C) (I)

$R^2$ is an alkyl of 1 to 4 carbon atoms;

Compounds A, B and I contain a chiral center—i.e. the 2-position of the benzodioxane nucleus. Accordingly, the compounds of the present invention may be prepared in either optically active form or as racemic mixtures. Unless otherwise specified, the compounds described herein are all in the racemic form. However, the scope of the subject invention herein is not to be considered limited to the racemic forms, but to encompass the individual optical isomers of the compounds.

If desired, the compound hereof may be resolved into its optical antipodes by conventional resolution means; for example by separation (e.g. fractional crystallization) of the diastereomeric salts formed by the reaction of the compound with optically active acids. Exemplary of such optically active acids are the optically active forms of camphor-10-sulfonic acid, 2-bromo-camphor-π-sulfonic acid, camphoric acid, methoxyacetic acid, tartaric acid, malic acid, diacetyltartaric acid, pyrrolidine-π-carboxylic acid and the like. The separated pure diastereomeric salts may then be cleaved by standard means to afford the respective optical isomers of the compound of Formula I.

Isolation and purification of the compound and intermediate described can be effected, if desired, by any suitable separation or purification procedure such as, for example, filtration, extraction, crystallization, column chromatography, thin-layer chromatography or thick-layer chromatography, or a combination of these procedures. Specific illustrations of suitable separation and isolation procedures can be had by reference to the examples hereinbelow. However, other equivalent separation or isolation procedures could, of course, also be used.

The salt products are also isolated by conventional means. For example, the reaction mixtures may be evaporated to a dryness, and the salts can be further purified by conventional methods.

The compound of formula A, is prepared as described by Augstein, et al in *J. Med. Chem.,* 8: 446 (1965). The compound of formula C is either commercially available or easily prepared by means known to those in the art.

The conversion of the compound of formula A into the compound of formula B (step 1) is carried out by treating the compound of formula A with an excess of alcohol under acidic conditions at low temperature, in the range of −10°C to +10°C and in the absence or presence of an aprotic organic solvent, such as, for example, diethyl ether or tetrahydrofuran. A preferred temperature range is 1°C to 5°C; preferred alcohols are methanol, ethanol and isopropanol; a preferred solvent is diethyl ether and a preferred acid is anhydrous hydrochloric acid. This reaction mixture is allowed to stand at the aforementioned low temperature for several hours or days before being warmed to room temperature of 15° to 30°, and the crude product is permitted to precipitate out. The crude product is recovered and purified by conventional means.

In the succeeding condensation of the compound of formula B with the compound of formula C to form

the compound of formula I, approximately equilimolar amounts of the two reagents are dissolved in a suitable polar solvent such as, for example, methanol, ethanol, isopropanol, preferably ethanol, and heated to enhance the reaction, to temperatures in the range of room temperature to 100°C, preferably at the reflux temperature of the solvent. The mixture is heated for a period of about 10 minutes to 24 hours, preferably 3 to 4 hours, or until reaction is complete. The product of formula I is then recovered by acidifying the reaction mixture and extracting the hydrochloride salt.

The compound of formula I as prepared in this manner may be isolated either as the hydrochloride salt or other salt or as the free base. The compound isolated as the free base may optionally be converted into the corresponding salt; if isolated as a salt it may be converted into the free base form or into other salts by direct salt interchange.

Utility and administration

The compound of the invention has been shown to effect $\alpha_2$ blockade in pithed rats and accordingly are useful in the affecting physiological phenomena controlled by $\alpha_2$ receptors. Among these phenomena are blood pressure, platelet aggregation, and mood. These compounds are, therefore, useful in treating hypertension and depression and in inhibiting platelet aggregation in human beings.

Administration of the active compound and salts described herein can be via any of the accepted modes of administration for therapeutic agents which affect $\alpha_2$ receptors. These methods include oral, parenteral and otherwise systemic forms. The preferred method of administration is oral, except in those cases where the subject is unable to inject, by himself, any medication. In those instances it may be necessary to administer the composition intravenously.

Depending on the intended mode, the compositions may be in the form of solid, semi-solid or liquid dosage forms, such as, for example, tablets, suppositories, pills, capsules, powders, liquids, suspensions, or the like, preferably in unit dosage forms suitable for single administration of precise dosages. The compositions will include a conventional pharmaceutical carrier or excipient and the compound of Formula I or a pharmaceutically acceptable salt thereof and, in addition, may include other medicinal agents, pharmaceutical agents, carriers, adjuvants, etc.

The amount of active compound administered will of course, be dependent on the subject being treated, the severity of the affliction, the manner of administration and the judgment of the prescribing physician. However, an effective dosage is in the range of 0.1—10 mg/kg day, preferably 0.5—5 mg/kg/day. For an average 70 kg human, this would amount to 7—700 mg per day, or preferably 35—350 mg/day.

For solid compositions, conventional non-toxic solid carriers include, for example, pharmaceutical grades of mannitol, lactose, starch, magnesium stearate, sodium saccharin, talcum, cellulose, glucose, sucrose, magnesium carbonate, and the like may be used. The active compound as defined above may be formulated as suppositories using, for example, polyalkylene glycols, for example, propylene glycol, as the carrier. Liquid pharmaceutically administerable compositions can, for example, be prepared by dissolving, dispersing, etc. an active compound as defined above and optional pharmaceutical adjuvants in a carrier, such as, for example, water, saline, aqueous dextrose, glycerol, ethanol, and the like, to thereby form a solution or suspension. If desired, the pharmaceutical composition to be administered may also contain minor amounts of nontoxic auxiliary substances such as wetting or emulsifying agents, pH buffering agents and the like, for example, sodium acetate, sorbitan monolaurate, triethanolamine sodium acetate, triethanolamine oleate, etc. Actual methods of preparing such dosage forms are known, or will be apparent, to those skilled in this art; for example, see *Remington's Pharmaceutical Sciences,* Mack Publishing Company, Easton, Pennsylvania, 15th Edition, 1975. The composition or formulation to be administered will, in any event, contain a quantity of the active compound(s) in an amount effective to alleviate the symptoms of the subject being treated.

For oral administration, a pharmaceutically acceptable non-toxic composition is formed by the incorporation of any of the normally employed excipients, such as, for example pharmaceutical grades of mannitol, lactose, starch, magnesium stearate, sodium saccharin, talcum, cellulose, glucose, sucrose, magnesium, carbonate, and the like. Such compositions take the form of solutions, suspensions, tablets, pills, capsules, powders, sustained release formulations and the like. Such compositions may contain 10%—95% active ingredient, preferably 25—70%.

Parenteral administration is generally characterized by injection, either subcutaneously, intramuscularly or intravenously. Injectables can be prepared in conventional forms, either as liquid solutions or suspensions, solid forms suitable for solution or suspension in liquid prior to injection, or as emulsions. Suitable excipients are, for example, water, saline, dextrose, glycerol, ethanol or the like. In addition, if desired, the pharmaceutical compositions to be administered may also contain minor amounts of non-toxic auxiliary substances such as wetting or emulsifying agents, pH buffering agents and the like, such as for example, sodium acetate, sorbitan monolaurate, triethanolamine oleate, etc.

A more recently devised approach for parenteral administration employs the implantation of a slow-release or sustained-release system, such that a constant level of dosage is maintained. See, e.g., U.S. Patent No. 3,710,795.

The following examples serve to illustrate the invention.

Preparation

Preparation of ethyl (1,4-benzodioxan-2-yl)acetimidate hydrochloride

17.5 g (0.10 mole) 2-cyanomethyl-1,4-benzodioxane, prepared as described by Augstein, et al, *J. Med. Chem.* 8: 446 (1965), was dissolved in a mixture containing 7 g ethanol and 50 ml diethyl ether. 4.5 g (0.15 moles) of dry HCl was bubbled though the mixture, which was then capped. The mixture was allowed to stand at 5°C for 4 days, followed by 3 days at room temperature. The crude product imidate hydrochloride (IV) precipitated out and was harvested by filtration, and washed with 100 ml ether, followed by 3×100 ml portions of methylene chloride. The solid was then purified by thin layer chromatography using 10% methanol in chloroform as a developing solvent. The product has an $R_f$ value of 0.7—0.8; starting material moves farther in this solvent system, and none was present in the crude product. The yield of product was 15.3 g, as the hydrochloride, or 59% yield.

Example 1

Conversion of Ethyl(1,4-benzodioxan-2-yl)acetimidate · HCl to 2-(1,4-benzodioxan-2-ylmethyl)imidazoline hydrochloride

Ethylenediamine (3.02 g) and the imidate salt prepared in Preparation 1 (10 g) in 100 ml of ethanol are refluxed for 10 hours. The mixture is evaporated and the residue is dissolved in 5% aqueous hydrochloric acid and washed with ether. After neutralization with ammonium hydroxide, the aqueous layer is extracted with ether and the ether is dried and evaporated to afford the free base as a foam. The hydrochloride salt is prepared by dissolving the base in methanolic-HCl and adding ether until crystallization occurs, m.p. 235—236°C.

Example 2

Conversion of free base to salt

Excess 3% hydrogen chloride in methanol is added to a solution of 1.0 g. 2 - (1,4 - benzodioxan - 2 - ylmethyl)imidazoline in 20 ml methanol. Diethyl ether is added until precipitation is complete. The product is filtered, washed with ether, air dried and recrystallized to give 2 - (1,4 - benzodioxan - 2 - ylmethyl)imidazoline · HCl.

In a similar manner, all compounds of Formula I in free base form may be converted to the acid addition salts by treatment with the appropriate acid, for example, hydrochloric acid, hydrobromic acid, sulfuric acid, nitric acid, phosphoric acid, acetic acid, propionic acid, glycolic acid, pyruvic acid, oxalic acid, malonic acid, succinic acid, malic acid, maleic acid, fumaric acid, tartaric acid, citric acid, benzoic acid, cinnamic acid, mandelic acid, methanesulfonic acid, ethanesulfonic acid, p-toluenesulfonic acid, and the like.

Example 3

Conversion of salt to free base

1.0 g of 2 - (1,4 - benzodioxan - 2 - ylmethyl)imidazoline · 2HCl suspended in 50 ml of ether is stirred with excess dilute aqueous potassium carbonate solution until the salt is completely dissolved. The organic layer is then separated, washed twice with water, dried over magnesium sulfate and evaporated to yield 2 - (1,4 - benzodioxan - 2 - ylmethyl)imidazoline as the free base.

Example 4

Directed interchange of acid addition salts 2 - (1,4 - benzodioxan - 2 - ylmethyl)imidazoline

2 - (1,4 - benzodioxan - 2 - ylmethyl)imidazoline acetate (1.0 g) is dissolved in a solution of 1 ml 50% aqueous sulfuric acid in 10 ml ethanol, and the solution evaporated to dryness. The product is suspended in ethanol and filtered, air dried and recrystallized from methanol/acetone to yield 2 - (1,4 - benzodioxan - 2 - ylmethyl)imidazoline: $HSO_4$.

Example 5

| Ingredients | Quantity per tablet, mgs. |
|---|---|
| Active ingredient | 25 |
| cornstarch | 20 |
| lactose, spray-dried | 153 |
| magnesium stearate | 2 |

The above ingredients are thoroughly mixed and pressed into single scored tablets.

Example 6

| Ingredients | Quantity, per tablet, mgs. |
|---|---|
| Active ingredient | 100 |
| lactose, spray-dried | 148 |
| magnesium stearate | 2 |

The above ingredients are mixed and introduced into a hard-shell gelatin capsule.

Example 7

| Ingredients | Quantity per tablet, mgs. |
|---|---|
| Active ingredient | 1 |
| cornstarch | 50 |
| lactose | 145 |
| magnesium stearate | 5 |

The above ingredients are mixed intimately and pressed into single scored tablets.

Example 8

| Ingredients | Quantity per tablet, mgs. |
|---|---|
| Active ingredient | 108 |
| lactose | 15 |
| cornstarch | 25 |
| magnesium stearate | 2 |

The above ingredients are mixed and introduced into a hard-shell gelatin capsule.

Example 9

| Ingredients | Quantity per tablet, mgs. |
|---|---|
| Active ingredient | 150 |
| lactose | 92 |

The above ingredients are mixed and introduced into a hard-shell gelatin capsule.

Example 10

An injectable preparation buffered to a pH of 7 is prepared having the following composition:

| Ingredients | |
|---|---|
| Active ingredient | 0.2 g |
| $KH_2PO_4$ buffer (0.4 M solution) | 2 ml |
| KOH (1 N) | q.s. to pH 7 |
| water (distilled, sterile) | q.s. to 20 ml |

Example 11

An oral suspension is prepared having the following composition:

| Ingredients | |
|---|---|
| Active ingredient | 0.1 g |
| fumaric acid | 0.5 g |
| sodium chloride | 2.0 g |
| methyl paraben | 0.1 g |
| granulated sugar | 25.5 g |
| sorbitol (70% solution) | 12.85 g |
| Veegum K (Vanderbilt Co.) | 1.0 g |
| flavoring | 0.035 ml |
| colorings | 0.5 mg |
| distilled water | q.s. to 100 ml |

Example 12

Characterisation of the alpha-adrenoceptor affinity of 2 - (1,4 - benzodioxan - 2 - ylmethyl)imidazoline

Materials and methods

Rat, isolated, transversely bisected vas deferens

Vasa deferentia were removed from male (200—300 g) SD(CD)SPF rats (Charles River, UK, Ltd.) and placed in a petri dish containing oxygenated Krebs' bicarbonate solution (millimolar concentrations): NaCl, 119; KCl, 4.7; $MgSO_4\ 7H_2O$, 1.0; $KH_2PO_4 \cdot 2H_2O$, 1.2; $CaCl_2 \cdot 6H_2O$, 2.5; $NaHCO_3$, 25.0; glucose, 11.0. Connective tissue was removed, and the vasa were transversely bisected. Prostatic portions, 12 mm in length, and epididymal portions, 14 mm in length, were prepared. Stainless steel threads (40 µm diameter) were tied through the walls of the lumen at each end of the bisected portions and the tissues were mounted under 0.5 g tension in 30 ml (prostatic portions) or 10 ml (epididymal portions) organ baths containing oxygenated Krebs' bicarbonate solution bathing one tissue whilst the contralateral tissue served as a control. Experiments to determine $\alpha_1$- and $\alpha_2$-adrenoceptor antagonist affinity were commenced after a 45 min equilibration period.

$\alpha_1$-Adrenoceptor affinity

Post-junctional a $\alpha_1$-adrenoceptor affinity was determined by constructing concentration response curves to amidephrine on the paired epididymal portions of vasa deferentia. Each concentration of amidephrine was allowed to act for 45 sec, or until a maximal response was obtained, before replacing the bathing fluid. A 6 min dose cycle was used, the Krebs' bicarbonate solution being replaced four times between additions. Responses for amidephrine were expressed as a percentage of the maximal response obtained in the control tissue. The potency of the antagonists was calculated using the method of Arunlakshana and Schild (1).

$\alpha_2$-Adrenoceptor affinity

Prejunctional $\alpha_2$-adrenoceptor affinity was determined using paired prostatic portions of vasa deferentia. Responses were elicited from contralateral prostatic portions of vasa deferentia by single pulse nerve stimulation (0.3 msec duration, 15V, 150 mA) every 5 min using a stimulator and a pulse power amplifier. After the 45 min equilibration period, full cumulative dose-inhibitory curves to xylazine were obtained. Results were expressed as a percentage of the maximal inhibition of the response to single pulse nerve stimulation obtained in the control tissue. The potency of the antagonists was calculated using the method of Arunlakshana and Schild (1).

Results

| Compound | $pA_2$ ($\alpha_2$) | $pA_2$ ($\alpha_1$) | Selectivity ratio $\alpha_2/\alpha_1$* |
|---|---|---|---|
| 2-(1,4-benzodioxan-2-ylmethyl)imidazoline | 7.0 | 5.0 | 100.0 |

* Selectivity is the antilog of the difference between the $pA_2$ values at $\alpha_2$- and $\alpha_1$-adrenoceptors.

These results indicate that 2 - (1,4 - benzodioxan - 2 - ylmethyl)imidazoline is a selective $\alpha_2$-antagonist.

Reference

1. Arunlakshana, O., and Schild, H.O.: Some quantitative uses of drug antagonists. Br. J. Pharmacol. *14*:48—58, 1959.

Example 13

Activity of 2-(1,4-benzodioxan-2-ylmethyl)imidazoline on systolic blood pressure of the spontaneously hypertensive rat

Materials and methods

Groups of four male SHR (Tac:N BR) of 350 to 450 g, previously matched for equivalent systolic blood pressures, were dosed, p.o., with 2 - (1,4 - benzodioxan - 2 - ylmethyl)imidazoline or vehicle at 9:00 a.m. on two consecutive days, but their blood pressures were recorded only on day 2. Each dose level of drug or vehicle was randomly assigned to a given group.

Immediately after dosing on the day of blood pressure measurement, SHR were put into restrainers and placed in a heated chamber (30.0°C±1.0°C) for 4 hr. Systolic blood pressures were recorded using photoelectric transducers. The coccygeal arteries of three rats were simultaneously occluded by tail cuffs that were automatically inflated by a pump to 300 mmHg and then deflated. A calibrated pressure curve and tail pulses were simultaneously monitored on an MFE recorder. Systolic blood pressure was considered to be the pressure at the appearance of the first pulse. Four consecutive traces (at 30 sec intervals) were recorded for each rat at 1, 2, 3 and 4 hr post-drug administration.

The mean systolic blood pressure of each rat, at each observation time, was calculated. The mean systolic pressure of the three to six animals on a given drug dosage group was then calculated at each observation time and compared to the mean systolic blood pressure of the control (vehicle only) group using a one-tailed student's "t" test. A dose of a compound was considered to exhibit significant antihypertensive activity at a given observation time when $p \leq 0.05$.

Drugs

In each study, one group of four SHR received the vehicle (solution of water and 0.4% polysorbate 80). All dose levels of 2 - (1,4 - benzodioxan - 2 - ylmethyl)imidazoline were administered as solutions in the water/polysorbate 80 vehicle.

| | | Hour 1 | | Hour 2 | | Hour 3 | | Hour 4 | |
|---|---|---|---|---|---|---|---|---|---|
| Study | Dose (mg/kg) | S.B.P. (% ↓) | ΔmmHg | S.B.P. (% ↓) | ΔmmHg | S.B.P. (% ↓) | ΔmmHg | S.B.P. (% ↓) | ΔmmHg |
| 1 | 50 | *32 | −77 | *31 | −77 | *16 | −36 | *25 | −60 |
| 2 | 50 | 2 | −4 | 2 | −3 | −3 | +7 | 0.5 | −1 |
| | 25 | −4 | +7 | −9 | +18 | *12 | −25 | 2 | −5 |
| 3 | 25 | −13° | +23 | *−19 | +36 | −*12 | +25 | −12 | +24 |
| | 12.5 | −15 | +26 | −6 | +11 | 1 | −2 | −0.5 | +1 |
| | 6.25 | −12 | +21 | 5 | −8 | 1 | −3 | 1 | −2 |

* Statistically significant $p \leq 0.05$

Studies on SHR show equivocal results concerning the effect of 2 - [1,4 - (benzodioxan - 2 - ylmethyl)imidazoline on blood pressure. Nevertheless, it is conceivable that 2 - [1,4 - benzodioxan - 2 - ylmethyl]imidazoline can lower blood pressure in other mammals.

**Claims for the Contracting States: BE CH DE FR GB IT LI LU NL SE**

1. 2-(1,4-benzodioxan-2-ylmethyl)imidazoline of the following formula

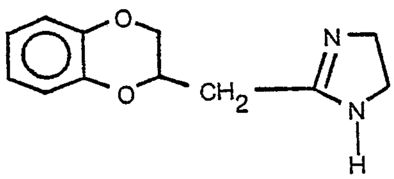

(I)

and the pharmaceutically acceptable acid addition salts thereof.

2. A pharmaceutical composition which comprises a therapeutically effective amount of the compound of Claim 1 or a pharmaceutically acceptable acid addition salt thereof in admixture with a pharmaceutically acceptable excipient.

3. A compound of Claim 1 or a pharmaceutical composition of Claim 2, for use in treating hypertension in mammals.

4. A compound of Claim 1 or a pharmaceutical composition of Claim 2, for use in treating depression of mammals.

5. A compound of Claim 1 or a pharmaceutical composition of Claim 2 for use in inhibiting blood platelet aggregation in mammals.

6. A process for preparing 2 - (1,4 - benzodioxan - 2 - ylmethyl)imidazoline of the following formula

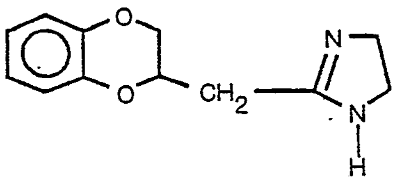

(I)

and the pharmaceutically acceptable acid addition salts thereof, which comprises:

(a) Condensing alkyl (1,4-benzodioxan-2-ylmethyl)acid imidate of the following formula

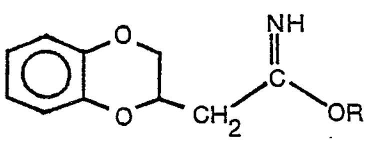

wherein R is lower alkyl of 1—4 carbon atoms; with ethylenediamine of the following formula:

$$H_2NCH_2CH_2NH_2$$

or;

(b) converting the free base of the compound of Formula I to a pharmaceutically acceptable acid addition salt; or

(c) converting a salt of the compound of Formula I to the free base; or

(d) converting a salt of the compound of Formula I to another salt.

7. A process according to Claim 6 wherein the compound prepared in accordance with Claim 6 is mixed with a pharmaceutically acceptable carrier.

8. The use of the compound of Claim 1 in the preparation of a pharmaceutical composition.

**Claims for the Contracting State: AT**

1. A process for preparing 2 - (1,4 - benzodioxan - 2 - ylmethyl)imidazoline of the following formula

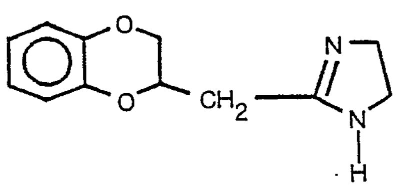

(I)

and the pharmaceutically acceptable acid addition salts thereof, which comprises:

(a) Condensing alkyl (1,4 - benzodioxan - 2 - ylmethyl)acid imidate of the following formula

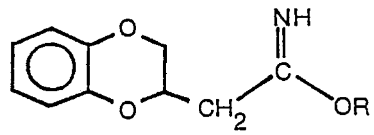

wherein R is lower alkyl of 1—4 carbon atoms; with ethylenediamine of the following formula:

$$H_2NCH_2CH_2NH_2$$

or;

(b) converting the free base of the compound of Formula I to a pharmaceutically acceptable acid addition salt; or

(c) converting a salt of the compound of Formula I to the free base; or

(d) converting a salt of the compound of Formula I to another salt.

2. A process according to Claim 1 wherein the compound prepared according to Claim 1 is mixed with a pharmaceutically acceptable carrier.

3. A process according to Claim 1 wherein the compound prepared according to Claim 1 is used in the preparation of a pharmaceutical composition.

4. A process according to Claim 1 or Claim 2 wherein there is prepared a composition for use in treating hypertension in mammals.

5. A process according to Claim 1 or Claim 2 wherein there is prepared a composition for use in treating depression in mammals.

6. A process according to Claim 1 or Claim 2 wherein there is prepared a composition for use in inhibiting blood platelet aggregation in mammals.

7. A process for preparing a pharmaceutical composition comprising mixing 2 - (1,4 - benzodioxan - 2 - ylmethyl)imidazoline of the formula

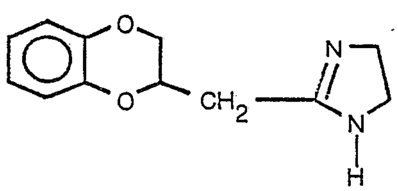

or a pharmaceutically acceptable acid addition salt thereof with a pharmaceutically acceptable carrier.

8. The use of 2 - (1,4 - benzodioxan - 2 - ylmethyl)imidazoline of the formula

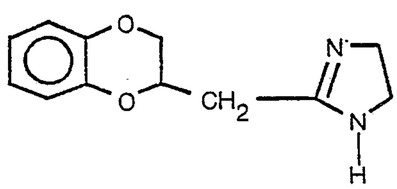

or a pharmaceutically acceptable salt thereof in the preparation of a pharmaceutical composition.

**Patentansprüche für die Vertragsstaaten: BE CH DE FR GB IT LI LU NL SE**

1. 2-(1,4-Benzodioxan-2-ylmethyl)imidazolin der folgenden Formel

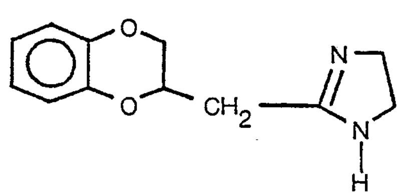

(I)

und die pharmazeutisch annehmbaren Säureadditionssalze desselben.

2. Pharmazeutische Zusammensetzung, die eine therapeutisch wirksame Menge der Verbindung gemäß Anspruch 1 oder eines pharmazeutisch annehmbaren Säureadditionssalzes derselben in Mischung mit einem pharmazeutisch annehmbaren Streckmittel umfaßt.

3. Eine Verbindung gemäß Anspruch 1 oder eine pharmazeutische Zusammensetzung gemäß Anspruch 2 zur Verwendung bei der Behandlung der Hypertension bei Mensch und Säugetier.

4. Eine Verbindung gemäß Anspruch 1 oder eine pharmazeutische Zusammensetzung gemäß Anspruch 2 zur Verwendung bei der Behandlung der Depression bei Mensch und Säugetier.

5. Eine Verbindung gemäß Anspruch 1 oder eine pharmazeutische Zusammensetzung gemäß Anspruch 2 zur Verwendung bei der Inhibierung der Thrombocytenaggregation bei Mensch und Säugetier.

6. Verfahren zur Herstellung von 2 - (1,4 - Benzodioxan - 2 - ylmethyl)imidazolin der folgenden Formel

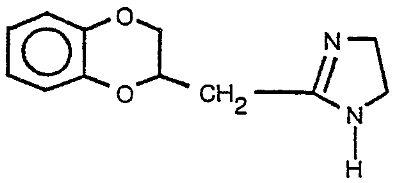

(I)

und der pharmazeutisch annehmbaren Säureadditionssalze desselben, dadurch gekennzeichnet, daß man

(a) Alkyl - (1,4 - benzodioxan - 2 - yl - methyl)säureimidat der folgenden Formel

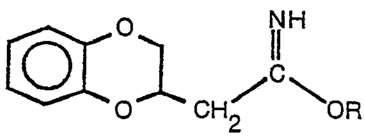

worin R niedrig Alkyl mit 1 bis 4 Kohlenstoffatomen ist, mit Ethylendiamin der folgenden Formel

$$H_2NCH_2CH_2NH_2$$

kondensiert, oder

(b) die freie Base der Verbindung der Formel I in ein pharmazeutisch annehmbares Säureadditionssalz umwandelt, oder

(c) ein Salz der Verbindung der Formel I in die freie Base umwandelt, oder

(d) ein Salz der Verbindung der Formel I in ein anderes Salz umwandelt.

7. Verfahren nach Anspruch 6, in welchem die gemäß Anspruch 6 hergestellte Verbindung mit einem pharmazeutisch annehmbaren Träger (Streckmittel) gemischt wird.

8. Die Verwendung der Verbindung gemäß Anspruch 1 bei der Herstellung einer pharmazeutischen Zusammensetzung.

**Patentansprüche für den Vertragsstaat: AT**

1. Verfahren zur Herstellung von 2 - (1,4 - Benzodioxan - 2 - yl - methyl)imidazolin der folgenden Formel

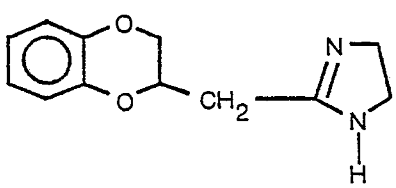

(I)

und der pharmazeutisch annehmbaren Säureadditionssalze desselben, dadurch gekennzeichnet, daß man

(a) Alkyl - (1,4 - benzodioxan - 2 - yl - methyl)säureimidat der folgenden Formel

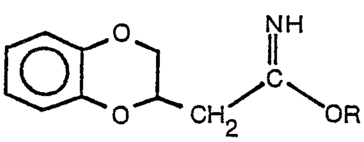

worin R niedrig Alkyl mit 1 bis 4 Köhlenstoffatomen ist, mit Ethylendiamin der folgenden Formel

$$H_2NCH_2CH_2NH_2$$

kondensiert, oder

(b) die freie Base der Verbindung der Formel I in ein pharmazeutisch annehmbares Säureadditionssalz umwandelt, oder

(c) ein Salz der Verbindung der Formel I in die freie Base umwandelt, oder

(d) ein Salz der Verbindung der Formel I in ein anderes Salz umwandelt.

2. Verfahren nach Anspruch 1, in welchem die gemäß Anspruch 1 hergestellte Verbindung mit einem pharmazeutisch annehmbaren Träger (Streckmittel) gemischt wird.

3. Verfahren nach Anspruch 1, in welchem die gemäß Anspruch 1 hergestellte Verbindung zur Herstellung einer pharmazeutischen Zusammensetzung verwendet wird.

4. Verfahren nach Anspruch 1 oder 2, in welchem man eine Zusammensetzung zur Verwendung bei der Behandlung der Hypertension bei Mensch und Säugetier herstellt.

5. Verfahren nach Anspruch 1 oder 2, in welchem man eine Zusammensetzung zur Verwendung bei der Behandlung der Depression bei Mensch und Säugetier herstellt.

6. Verfahren nach Anspruch 1 oder 2, in welchem man eine Zusammensetzung zur Verwendung bie der Inhibierung der Thrombocytenaggregation bei Mensch und Säugetier herstellt.

7. Verfahren zur Herstellung einer pharmazeutischen Zusammensetzung, dadurch gekennzeichnet, daß man 2 - (1,4 - Benzodioxan - 2 - yl - methyl)imidazolin der Formel

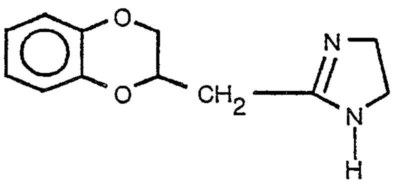

oder ein pharmazeutisch annehmbares Säureadditionssalz desselben mit einem pharmazeutisch annehmbaren Träger mischt.

8. Die Verwendung von 2 - (1,4 - Benzodioxan - 2 - yl - methyl)imidazolin der Formel

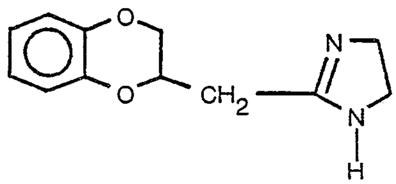

oder eines pharmazeutisch annehmbaren Salzes desselben bei der Herstellung einer pharmazeutischen Zusammensetzung.

**Revendications pour les Etats Contractants: BE CH DE FR GB IT LI LU NL SE**

1. Le (1,4 - benzodioxanne - 2 - yl - méthyl) - imidazoline de formule suivante:

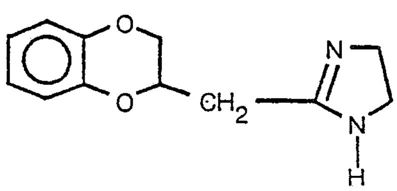

(I)

et ses sels d'addition d'acides pharmaceutiquement acceptables.

2. Composition pharmaceutique, qui comprend une quantité à effet thérapeutique du composé suivant la revendication 1 ou d'un sel d'addition d'acide pharmaceutiquement acceptable de ce composé en mélange avec un excipient acceptable du point de vue pharmaceutique.

3. Composé suivant la revendication 1 ou composition pharmaceutique suivant la revendication 2, destinés à être utilisés dans le traitement de l'hypertension chez des mammifères.

4. Composé suivant la revendication 1 ou composition pharmaceutique suivant la revendication 2, destinés à être utilisés dans le traitement d'un état de dépression chez des mammifères.

5. Composé suivant la revendication 1 ou composition pharmaceutique suivant la revendication 2, destinés à être utilisés pour inhiber l'agrégation des plaquettes sanguines chez les mammifères.

6. Procédé de préparation de la 2 - (1,4 - benzodioxanne - 2 - yl - méthyl) - imidazoline de formule suivante:

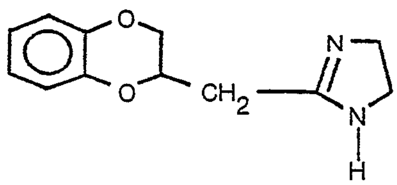

(I)

et de ses sels d'addition d'acides pharmaceutiquement acceptables, qui consiste:

(a) à condenser un (1,4 - benzodioxanne - 2 - yl - méthyl) - imidate d'alkyle de formule suivante:

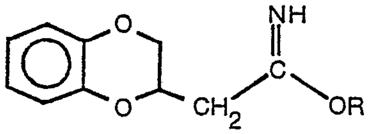

dans laquelle R est un radical alkyle inférieur ayant 1 à 4 atomes de carbone; avec l'éthylène-diamine de formule suivante:

$$H_2NCH_2CH_2NH_2$$

ou bien

(b) à convertir la base libre du composé de formule I en un sel d'addition d'acide pharmaceutiquement acceptable; ou bien

(c) à convertir un sel du composé de formule I en la base libre; ou bien

(d) à convertir un sel du composé de formule I en un autre sel.

7. Procédé suivant la revendication 6, dans lequel le composé préparé conformément à la revendication 6 est mélangé avec un support acceptable du point de vue pharmaceutique.

8. Utilisation du composé suivant la revendication 1 dans la préparation d'une composition pharmaceutique.

**Revendications pour l'Etat Contractant: AT**

1. Procédé de préparation de la 2 - (1,4 - benzodioxanne - 2 - yl - méthyl) - imidazoline de formule suivante:

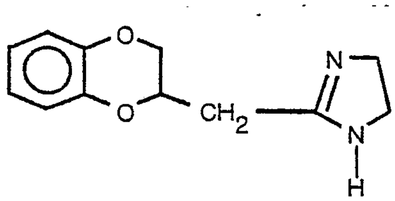

(I)

et de ses sels d'addition d'acides pharmaceutiquement acceptables, qui consiste:

(a) à condenser un (1,4 - benzodioxanne - 2 - ylméthyl)imidate d'alkyle de formule suivante:

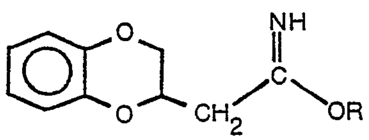

dans laquelle R est un radical alkyle inférieur ayant 1 à 4 atomes de carbone; avec l'éthylène-diamine de formule suivante:

$$H_2NCH_2CH_2NH_2$$

ou bien

(b) à convertir la base libre du composé de formule I en un sel d'addition d'acide pharmaceutiquement acceptable; ou bien

(c) à convertir un sel du composé de formule I en la base libre; ou bien

(d) à convertir un sel du composé de formule I en un autre sel.

2. Procédé suivant la revendication 1, dans lequel le composé préparé conformément à la revendication 1 est mélangé avec un support pharmaceutiquement acceptable.

3. Procédé suivant la revendication 1, dans lequel le composé préparé conformément à la revendication 1 est utilisé dans la préparation d'une composition pharmaceutique.

4. Procédé suivant la revendication 1 ou la revendication 2, dans lequel est préparée une composition destinée à être utilisée dans le traitement de l'hypertension chez les mammifères.

5. Procédé suivant la revendication 1 ou la revendication 2, dans lequel est préparée une composition destinée à être utilisée pour le traitement d'un état de dépression chez les mammifères.

6. Procédé suivant la revendication 1 ou la revendication 2, dans lequel es préparée une composition destinée à être utilisée pour inhiber l'agrégation des plaquettes sanguines chez les mammifères.

7. Procédé de préparation d'une composition pharmaceutique, qui consiste à mélanger de la 2 - (1,4 - benzodioxanne - 2 - yl - méthyl) - imidazoline de formule

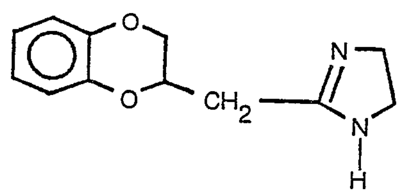

ou un sel d'addition d'acide pharmaceutiquement acceptable de ce composé avec un support acceptable du point de vue pharmaceutique.

8. Utilisation de la 2 - (1,4 - benzodioxanne - 2 - yl - méthyl) - imidazoline de formule

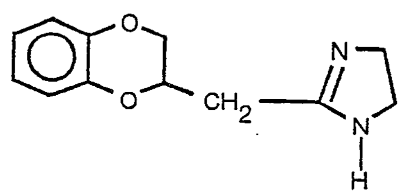

ou d'un sel pharmaceutiquement acceptable de ce composé dans la préparation d'une composition pharmaceutique.